Europäisches Patentamt

European Patent Office

Office européen des brevets

(19)

(11) Veröffentlichungsnummer: **0 090 343**
A2

# (12) EUROPÄISCHE PATENTANMELDUNG

(21) Anmeldenummer: **83102870.9**

(22) Anmeldetag: **23.03.83**

(51) Int. Cl.³: **C 12 N 5/00**, C 12 N 15/00, C 12 P 1/00 // C12R1/91, G01N33/54, A61K39/395

(30) Priorität: **27.03.82 DE 3211356**

(43) Veröffentlichungstag der Anmeldung: **05.10.83** Patentblatt 83/40

(84) Benannte Vertragsstaaten: **AT BE CH DE FR GB IT LI LU NL SE**

(71) Anmelder: **BEHRINGWERKE Aktiengesellschaft, Postfach 1140, D-3550 Marburg 1 (DE)**

(72) Erfinder: **Bosslet, Klaus, Dr., Ockershäuser Allee 5, D-3550 Marburg 1 (DE)**
Erfinder: **Kurrle, Roland, Dr., Schenkendorfweg 18, D-3550 Marburg 1 (DE)**
Erfinder: **Sedlacek, Hans-Harald, Dr., Am Sonnenhang 3, D-3550 Marburg 1 (DE)**
Erfinder: **Ax, Wolfgang, Prof. Dr., Am Kornacker 76, D-3550 Marburg 6 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) Zell-Hybrid, Verfahren zu seiner Herstellung sowie seine Verwendung.

(57) Es wird ein Zellhybrid beschrieben, das monoklonale Antikörper sekretiert, die mit einem Bestandteil tierischer Zellen reagieren, sowie ein Verfahren zur Herstellung dieses Hybrids. Die Antikörper können als Diagnostika sowie zur Therapie von Tumorerkrankungen verwendet werden.

EP 0 090 343 A2

BEHRINGWERKE AKTIENGESELLSCHAFT   Hoe 82/B006
Dr.Ha/Bl.

0090343

Zell-Hybrid, Verfahren zu seiner Herstellung sowie

seine Verwendung

Die Erfindung betrifft ein Zellhybrid, das monoklonale Antikörper sekretiert, die mit Bestandteilen tierischer Zellen reagieren, ein Verfahren zur Herstellung des Hybrids sowie seine Verwendung zur Herstellung dieser monoklonalen Antikörper. Diese Antikörper können als Diagnostika sowie zur Therapie von Tumorerkrankungen verwendet werden.

Die Technik der Fusion von Zellen des Immunsystems mit Tumorzellen ist in Nature 256, 495, 1975 beschrieben. Prinzipiell besteht die Technik darin, daß ein Antikörper sezernierender B-Lymphozyt mit einer Tumorzelle vereinigt wird, wobei das gebildete Hybrid die Eigenschaften beider Zellen behält; von der Tumorzelle die Fähigkeit der unbegrenzten Teilungsfähigkeit und vom B-Lymphozyten die Eigenschaft Antikörpermoleküle eines Typs zu sekretieren. Somit ist es möglich, die Antikörperantwort eines Organismus gegen ein bestimmtes Immunogen auf Dauer zu verselbständigen und die besonders interessanten monoklonalen Antikörper durch permanentes Kultivieren der sie sekretierenden Hybride herzustellen.

In Proc. Nat. Acad. Sci. U.S.A., Vol. 78, 3, 1695-1699, (1981) ist ein nach diesem Verfahren hergestellter Antikörper beschrieben, der das im Zellkern nur von proliferierenden Zellen des Menschen befindliche Protein (p53) immunpräzipitiert.

Es wurden auch bereits monoklonale Antikörper generiert, die mit Membranantigenen auf menschlichen Melanomen und Colon-Carcinom reagieren (Proc. Nat. Acad. Sci. U.S.A. (1979) 76, 3, 1438 und (1980) 77, 4, 2138). In Br. J. Cancer 43, 696, (1981) werden monoklonale Antikörper beschrieben, die durch Fusion von humanen B-Lymphozyten mit Ratten oder Mausmyelomen erzeugt wurden. Diese Hybride waren nicht stabil und stellten 10 Wochen nach der Fusion die Antikörperproduktion ein. Es wurde eine Reaktivität mit der Zellmembran von 4 Lungentumoren und keine Reaktivität mit je einer normalen Lungenprobe, das heißt auch nicht mit Kernen, mit normalen Lymphozyten, Erythrozyten und normalem Colon nachgewiesen.

In J. of surgical research 30, 403, (1981) werden zur Generation von monoklonalen Antikörpern Maus-B-Zellen mit Mausmyelomzellen fusioniert. Es wurden Hybridome erhalten, deren Produkte Reaktivität mit Zellmembranen von einem von sieben Lungen-Carcinomen und einem von zweien Colon-Carcinomen, mit dreien von sieben Lungen-Carcinomen, zwei Colon- und zwei Mamma-Carcinomen sowie mit sieben Lungen-, drei Mamma- und zwei Colon-Carcinomen, vier Melanomen und einer embryonalen Zellinie vom Menschen zeigten. Keine Reaktivität wurde mit humanen Lymphoblasten und peripheren Blutlymphozyten beobachtet, auch nicht mit deren Kerner

Überraschenderweise haben wir nun gefunden, daß man Hybridome erzeugen kann, die monoklonale Antikörper sezernieren, die sich spezifisch an einen wasserlöslichen antigenen Bestandteil der Zellkerne aller getesteten Gewebe(Tab. 1) verschiedener Säugetier-Species, darunter Mensch, Ratte und Hamster, binden.

Weiterhin wurden Hybridome hergestellt, die Antikörper ausscheiden, die sich an Zellmembranen von humanen Lungentumorzellen und an Zellmembranen von embryonalen Fibroblasten binden (siehe Tabelle 1) und damit ein anderes Reaktivitätsspektrum als die bislang beschriebenen Antikörper aufweisen.

Gegenstand der Erfindung sind demnach Hybridome, die monoklonale Antikörper ausscheiden, die spezifisch mit Kernen von allen Säugetierzellen reagieren.

Gegenstand der Erfindung sind weiterhin Hybridome, die Antikörper ausscheiden, die mit humanen Lungencarcinomzellen und embryonalen Fibroblasten, nicht jedoch mit normalem Lungengewebe, Lungenfibroblasten und einer Reihe von anderen humanen Tumoren reagieren.

Gegenstand der Erfindung ist weiterhin ein Verfahren zur Herstellung dieser Hybridome sowie ihre Verwendung zur Herstellung von Antikörpern und deren Verwendung zu diagnostischen Zwecken.

In diesem Verfahren werden Tiere mit Zellen der Calu-1-Plattenepithelcarcinomzellinie nach an sich bekannten Verfahren immunisiert, B-Lymphozyten aus den Tieren gewonnen und mit an sich bekannten Tumorzellen fusioniert. Zur Immunisierung von höheren Tieren, bevorzugt Säuger, besonders bevorzugt Mäuse, Ratten, Kaninchen, Schaf und Hamster, wurde die Calu-1-Plattenepithelcarcinomzellinie benutzt. Diese Zellinie kann vom Laboratory of Immuno Diagnosis, NCI, National Institutes of Health, Public Health Service, U.S.Department of Health, Education and Welfare, Bethesda, Md. 20 014, erhalten werden. Sie ist beschrieben in J.Natl.Cancer Inst. $\underline{59}$, 1413-18 (1977).

B-Lymphozyten enthaltende Organe der immunisierten Organismen werden aseptisch entnommen und mit solchen Tumorzellen fusioniert, die aufgrund einer Mutation in Anwesenheit im Stand der Technik bekannter chemischer Verbindungen im Kulturmedium, einem sogenannten Selektionskulturmedium (beispielsweise Science (1964) $\underline{145}$,709), nicht wachsen.

Der Fusionsvorgang wird nach an sich bekannten Verfahren durch Mischen der B-Lymphozyten und der Tumorzellen unter Zugabe eines bekannten Zellfusionsagens, vorzugsweise Polyäthylenglykol oder Sendaivirus, durchgeführt. Nach der Fusion werden die entstandenen Hybride in einem Selektionsgewebekulturmedium, das zur Schaffung optimaler Bedingungen mit Hypoxanthin, Aminopterin und Thymidin komplementiert ist, wachsen lassen, Hybridklone gewonnen und die Gewebekulturüberstände dieser Klone auf ihren Gehalt an spezifischem Antikörper getestet. Die Hybridklonüberstände können mittels eines spezifischen Radioimmunoassays (RIA), eines spezifischen Enzyme-linked-Immunsoadsorbens-Assays (ELISA), oder Immunfluorenszenztechniken (IF) an Zellmaterial, Extrakten oder gereinigten Molekülen auf spezifische Antikörper geprüft werden.

Die Hybride, deren Überstände gegen das zum Immunisieren verwendete Antigen spezifische Antikörper enthalten, werden nochmals kloniert und erneut nach obigen Methoden auf ihre Fähigkeit, spezfische Antikörper zu sekretieren, überprüft. Die Hybridzellen werden nach Abtrennung vom Überstand unter Bedingungen eingefroren, die ihre Rekultivierung nach Auftauen ermöglichen. Solche Einfriermethoden sind bekannt. Die Überstände werden auf Reaktivität mit anderen als dem spezifischen Antigen überprüft. Die sogenannte Feinspezifitätsanalyse der monoklonalen Antikörper wird ebenfalls mit RIA, ELISA oder IF-Techniken durchgeführt.

Die mit Hilfe dieser Hybridome gewonnenen monoklonalen Antikörper können als Diagnostika zum Erkennen von Autoimmunkrankheiten und zur Diagnose und Therapie von Tumoren und anderen Krankheiten verwendet werden.

Die Antikörper können weiterhin für therapeutische Zwecke als Transportmoleküle für Tumor-toxische Substanzen oder für toxische Substanzen dienen, welche mittels chemischer Methoden oder aber über Liposomen indirekt an die Antikörper gekoppelt sind.

- 5 -

Weiterhin können die Moleküle zur Tumorlokalisierung mit einer nachweisbaren Gruppe versehen werden, um als Immundiagnostika gebraucht zu werden.

Für die therapeutische Anwendung gegen Tumoren würden die Antikörper in einer Menge von 0.1 µg - 1 g/kg Körpergewicht pro Tag verabreicht.

0090343

Tabelle 1

Zelltypen, an denen die von den erfindungsgemäßen Hybridomen sekretierten Antikörper mittels Immunfluoreszenz getestet wurden:

Humane Tumorzellen

    5 Lungen Ca. (Zeilkultur)

    6 Lungen Ca. (Gewebe)

    3 Magen Ca. (Gewebe)

    1 Transversum Ca. (Gewebe)

    1 Rektum Ca. (Gewebe)

    1 Colon Ca. (Zellkultur)

    1 Schilddrüsen Ca. (Gewebe)

    1 Ovarial Ca.(Gewebe)

    1 Mamma Ca. (Gewebe)

    2 Melanom (Zellkultur)

    1 Melanom (Gewebe)

    1 Cervix Ca. (Zellkultur)

    1 Fibrosarkom(Zellkultur)

    1 Hypernephrom (Zellkultur)

    1 Hypernephrom (Gewebe)

    1 Burkitt's Lymphom (Zellkultur)

    2 EBV-Tranf.Lymphom (Zellkultur)

    1 Hodgkin's Lymphom (Zellkultur)

Humane Normalzellen von unterschiedlichen Spendern

    3 Humanfibroblasten Lunge (Zellkultur)

    3 Embryonale Fibroblasten (Zellkultur)

    6 Lunge normal (Gewebe)

Tierische Zellen

    1 Ratte (alle Organe) (Gewebe)

    1 R3230 (Zellkultur), ein Mammacarcinom der Ratte

      LS Lymphom Maus (Zellkultur)

     721 Lymphom Maus (Zellkultur)

    P815 Lymphom Maus (Zellkultur)

    Baby-Hamster-Kidney-Zelle (BHK;Zellkultur)

Die erfindungsgemäßen gegen Zellkernantigene gerichteten Antikörper reagieren mit den Zellkernen aller getesteten oben genannten Zelltypen und weiterhin mit den Zellkernen aller Organe des Hamsters und der Maus.

Tabelle 2

Ergebnisse der Austestung von Antikörpern (sekretiert von Hybridomen), die sich an die Zellmembranen humaner Lungentumoren binden:

| Monoklo- nale An- tikörper aus | Bindungen an | | | | | |
| --- | --- | --- | --- | --- | --- | --- |
| | Lungentumor | | andere Tumoren | Normalgewebe | | Xenogenes Gewebe |
| | Zellkultur | Schnitt | | embryonal | sonstig | |
| Hybrid A | + | + | – | + | – | – |
| Hybrid B | + | – | – | (+) | – | – |
| Hybrid C | + | – | – | + | – | – |
| Hybrid D | + | + | + | + | – | – |
| Hybrid E | + | + | + | – | + | – |
| Hybrid F | + | + | + | + | + | – |
| Hybrid G | + | + | + | – | – | – |

Diese Tabelle gibt das Spezifitätsspektrum der von sieben Hybridomen, die nach dem erfindungsgemäßen Verfahren erhalten wurden, sekretierten moleklonalen Antikörper wieder.

Beispiel

Zur Immunisierung von Balb/c Mäusen wurden $10^7$ Zellen einer etablierten Plattenepithelkarzinom-Zellinie der Lunge (CaLu-1, J. Nat. Cancer Inst. (1977) 59, 5, 1413-1418) nach Trypsinierung (0.25 % Trypsin) intraperitoneal an den Tagen 0 und 7 inokuliert. An Tag 11 wurden die Milzen der immunisierten Tiere aseptisch entnommen, mittels eines Siebes zerkleinert und in Kulturmedium (Dulbecco modified Eagle medium) ohne foetales Kälberserum (FKS) 2 x gewaschen, 5 Minuten bei 1800 UpM zentrifugiert und die Zahl der lebenden Lymphozyten mittels der Trypanblau Vitalitätsfärbung bestimmt.

Parallel hierzu wurden in Gewebekultur gehaltene Mausmyelomzellen vom Stamm X63-Ag8653 (J. Immunol. (1979), 123, 4, 1548) 2 x mit Kulturmedium ohne FKS gewaschen und die Zahl der lebenden Tumorzellen ermittelt (wie oben).

Zur Fusion wurden die Maus-Myelomzellen mit den Lymphozyten im Verhältnis 1 : 3 gemischt, zentrifugiert (5 min, 1800 UpM) und zum Pellet 0.5 ml 50 %ige PEG-Lösung in Wasserbad bei 37$^{\circ}$C gegeben und 1 Minute inkubiert. Unter leichtem Schütteln wurden die Verklumpungen zerschlagen und 4 - 5 ml HAT-Medium tropfenweise zugegeben und 2 - 4 Minuten stehen gelassen.

Nach einer 10-minütigen Zentrifugation bei 800 UpM wurde das Sediment so in HAT-Medium verdünnt, daß $5 \times 10^4 - 1 \times 10^6$ Zellen pro Loch einer 24 Loch Costar Platte ausgesät werden konnten. Nach 14 Tagen konnten die ersten wachsenden Hybridomaklone entnommen werden und jeder Klon in ein neues Loch einer 24 Loch-Platte transferiert werden. Nachdem die Hybridklone soweit herangewachsen waren, daß sie den Boden der Löcher total bedeckten, wurde von jedem Loch, das einen wachsenden Klon enthielt, 1 ml Überstand entnommen, 1 ml neues HAT-Medium zu den Zellen zugegeben und der Hybridomaüberstand auf spezifischen Antikörpergehalt überprüft. Hierzu wurden aus Gewebekultur entnommene trypsinierte Calu-1-Zellen in Terasaki-Platten ausgesät (1000 Zellen/Loch) und 24 Stunden später, nachdem sie in den Löchern der Platten festgewachsen waren, mit Hilfe einer indirekten Immunfluoreszenztechnik untersucht. Hierzu wurden die Platten mit PBS (phosphat-gepufferte isotonische Kochsalzlösung), welche $Mg^{++}$, $Ca^{++}$ und 0.1 % BSA enthält, 2 x gewaschen und 15 Minuten bei $4^{\circ}C$ mit dem zu testenden Überstand in einer feuchten Kammer inkubiert. Danach wurden die Terasaki Platten 3 x mit PBS gewaschen und mit einem FITC-markierten Kaninchen-Anti-Maus-Gamma-globulin 15 Minuten bei $4^{\circ}C$ in einer feuchten Kammer inkubiert. Anschließend wurden die Zellen 3 x mit PBS gewaschen und unter dem Fluoreszenzmikroskop untersucht. Eine zweite indirekte Immunfluoreszenztechnik an in flüssigem Stickstoff gelagertem Tumorgewebe wurde parallel zu der oben beschriebenen Technik durchgeführt. Hierzu wurde Tumorgewebe von etwa 0,5 cm Kantenlänge direkt aus dem Patienten oder als Xenotransplantat von nackten Mäusen auf Korkplättchen mit einem Tropfen PBS auf flüssigem Stickstoff schwimmend bei $-196^{\circ}C$ eingeforen und bei $-60^{\circ}C$ gelagert.

Zum Herstellen von Gefrierschnitten wurden die Korkplatten, auf denen die Tumorstückchen fixiert sind, mit Klebstoff auf Metallzylinder festgefroren. Anschließend wurden die Metallzylinder mit den entsprechenden Proben im Gefriermikrotom fixiert und Schnitte von 6 µm Dicke angefertigt.

Die Schnitte wurden auf Objektträger genommen, pro Schnitt 25/ul des zu testenden Antikörper-Überstandes zugegeben und 30 min in einer feuchten Kammer bei Raumtemperatur inkubiert. Nach dreimaligem Waschen in PBS mit 0,1 % BSA wurde der Objektträger mit einem Kaninchen-Anti-Maus Immunglobulin Antikörper, der FITC-markiert war, 30 Minuten bei Raumtemperatur in einer feuchten Kammer inkubiert.

Nach dreimaligem Waschen in PBS mit 0.1 % BSA wurden die Objektträger luftgetrocknet und im Dunkeln bis zur lichtmikroskopischen Untersuchung aufbewahrt. Für die Mikroskopie wurden die Objektträger mit Glycerin und Deckglas eingedeckt.

Überstände, die in einem der beiden Tests eine deutliche Reaktion bewirkten, wurden als positiv angesehen und einer Klonierung mittels eines Mikromanipulators unterzogen. Unter mikroskopischer Kontrolle wurde mit dieser Technik eine Hybridomzelle in eine Kapillare aufgezogen und in ein Loch einer 96 Loch Platte, die vorher mit $10^4$ Mauslymphozyten pro Loch als Nährzelle belegt wurden, ausgesät, wachsen lassen und die Überstände dieser Klone auf ihre Feinspezifität mittels der indirekten Immunfluoreszenzen an unterschiedlichen Geweben und an in vitro kultivierten Linien untersucht.

Es wurden Hybridomas erhalten, welche monoklonale Antikörper sekretieren, die mit Zellkernen spezifisch (Tab.1) reagieren.

Des weiteren werden Hybridomas erhalten, welche mit humanen Lungencarcinomzellen, schwach auch mit humanen embryonalen Fibroblasten, nicht jedoch mit humanen Lungenfibroblasten, normalem Lungengewebe und anderen

- 11 -

Tumoren (zum Beispiel Magencarcinom, Coloncarcinom, Hypernephrom, Melanom, Ovarialcarcinom, Mammacarcinom) und mit Normalgewebe von der Maus reagierten (siehe Tabelle I und II).

Verwendete Medien und Hilfsmittel

HAT-Selektivmedium: Science 145, p.709 (1964)

Medium für Mäuse-Myelomzellen (15 % FKS)

150 ml   Foetales Kälber-Serum (inaktiviert)

70 ml   $NaHCO_3$   5 %ig

20 ml   L-Glutamin   200 mM

10 ml   Antibiotica-Antimycotica (je 20 000 E Penicillin [*]

u.Streptomycin)

10 ml   Eagles-Dulbecco   10-fach

ad 1000 ml mit Aqua bidest. (675 ml)

Medium für Fusionen   (20 % FKS)

200 ml   Foetales Kälber-Serum (inaktiviert)

70 ml   $NaHCO_3$   5 %ig

20 ml   L-Glutamin   200 mM

10 ml   Antibiotica-Antimycotica   [*] siehe oben

70 ml   Eagles-Dulbecco   10-fach

ad 1000 ml mit Aqua bidest. (630 ml)

Medium ohne FKS

70 ml   $NaHCO_3$   5%ig

20 ml   L-Glutamin   200 mM

10 ml   Antibiotica-Antimycotica   [*] siehe oben

90 ml   Eagles-Dulbecco   10-fach

ad 1000 ml mit Aqua bidest. (810 ml)

Polyaethylenglykol für die Fusion

PEG   Fa. Merck   MG 4000   für die Gaschromatographie

20 g   PEG in einer Flasche 20 Minuten autoklavieren.

28 ml Dulbecco's PBS zugeben, solange PEG noch warm.

Patentansprüche:

1.  Zellhybrid, dadurch gekennzeichnet, daß es monoklonale Antikörper ausscheidet, die spezifisch mit einem wasserlöslichen antigenen Bestandteil der Kerne von Säugetierzellen reagieren.

2.  Zellhybrid, dadurch gekennzeichnet, daß es monoklonale Antikörper ausscheidet, die mit Zellmembranen humaner Lungencarcinomzellen und mit embryonalen Fibroblasten, nicht jedoch mit denen normaler humaner Lunge, mit Lungenfibroblasten und mit anderen humanen Tumoren reagieren.

3.  Zellhybrid nach Anspruch 1 oder 2, dadurch gekennzeichnet, daß es durch Fusionieren eines B-Lymphozyten aus einem mit der Calu-1-Plattenepithelcarcinomzelllinie immunisierten höheren Tier mit einer Tumorzelle erhalten wurde.

4.  Verfahren zur Herstellung eines Zellhybrids nach Anspruch 2, dadurch gekennzeichnet, daß ein höheres Tier mit Zellen der Calu-1-Plattenepithelcarcinomzellinie immunisiert wird, B-Lymphozyten aus diesem Tier gewonnen und mit einer Tumorzelle fusioniert werden.

5.  Verwendung eines Hybrids nach einem der Ansprüche 1 oder 2 zur Herstellung monoklonaler Antikörper, die spezifisch mit einem antigenen Bestandteil tierischer Zellkerne reagieren.

6.  Verwendung eines Hybrids nach einem der Ansprüche 1, 2 oder 3 zur Herstellung monoklonaler Antikörper, die mit Zellmembranen von Lungencarcinomzellen und mit embryonalen Fibroblasten, nicht jedoch mit Normallungengewebe und anderen Tumoren reagieren.